# EUROPEAN PATENT SPECIFICATION

(11) **EP 4 418 946 B1**
(45) Date of publication and mention of the grant of the patent: **02.12.2026**
(21) Application number: 22793800.8
(22) Date of filing: 12.10.2022
(51) Int. Cl.: A24F 40/60, A24F 7/00, A24F 40/40

(54) **AEROSOL PROVISION SYSTEM**
AEROSOLBEREITSTELLUNGSSYSTEM
SYSTÈME DE FOURNITURE D'AÉROSOL

(30) Priority: 22.10.2021 US 202117451846
(43) Date of publication of application: 28.08.2024
(60) Divisional application: 26214077.5
(73) Proprietor: Nicoventures Trading Limited, London WC2R 3LA (GB)
(72) Inventor: MOLONEY, Patrick, London WC2R 3LA (GB); LUKAN, Sean, Winston-Salem, North Carolina 27102 (US)
(74) Representative: Dehns
(86) International application number: PCT/GB2022/052589
(87) International publication number: WO 2023/067304

(56) References cited:
- EP-A1- 3 085 257
- EP-A1- 3 616 535
- WO-A1-2021/005367
- US-A- 3 084 698
- US-B1- 10 820 629

## Description

### TECHNICAL FIELD

The present disclosure relates to aerosol provision systems such as, but not limited to, nicotine delivery systems (e.g. electronic cigarettes and the like).

### BACKGROUND

Electronic aerosol provision systems often employ an electronic cigarette (e-cigarette) or more generally an aerosol provision device. Such an aerosol provision system typically contains aerosolizable material (also called aerosol-generating material), such as a reservoir of fluid or liquid containing a formulation, typically but not necessarily including nicotine, or a solid material such as a tobacco-based product, from which a vapor/aerosol is generated for inhalation by a user, for example through heat vaporization. Thus, an aerosol provision system will typically comprise a vaporizer (also called an aerosol generator), e.g. a heating element, arranged to aerosolize a portion of aerosolizable material to generate a vapor.

Once a vapor has been generated, the vapor may be passed through flavoring material to add flavor to the vapor (if the aerosolizable material was not itself flavored), after which the (flavored) vapor may be then delivered to a user via a mouthpiece from the aerosol provision system.

A potential drawback of existing aerosol provision systems and associated aerosol provision devices is that, particularly for those with a visual impairment, it can be challenging to discern particular characteristics or operations of aerosol provision systems, and/or any aerosol provision devices therefrom. Various approaches are therefore described herein which seek to help address or mitigate some of the issues discussed above, through the use of a thermal element on a mouthpiece of the aerosol provision system which can be useable to provide thermal feedback to the user, via their mouth, lips, or tongue.

Particularly for those with a visual impairment, it can also be difficult for the user to know exactly where their mouth and lips should be exactly located over the mouthpiece in use. Thus approaches described herein also seek to make it easier for such users to know exactly how best to locate their mouth and/or lips over the mouthpiece.
EP 3085257 A1 discloses an electronic smoking device.
EP 3616535 A1 discloses an aerosol generation method and apparatus.
US 10820629 B1 discloses tobacco product compositions and a delivery system.
WO 2021/005367 A1 discloses vapour delivery systems.
US 2084698 A discloses a connecting device.

### SUMMARY

According to a first aspect of certain embodiments there is provided a mouthpiece for an aerosol provision system, the mouthpiece comprising: at least one aerosol outlet channel for delivering an aerosol to a user of the mouthpiece, wherein the mouthpiece comprises at least one thermal element for controlling the temperature of a portion of the mouthpiece, wherein the portion comprises an exterior surface of the mouthpiece, wherein the thermal element comprises at least one of a heater and/or cooler

According to a second aspect of certain embodiments there is provided an aerosol provision system for generating an aerosol, comprising the mouthpiece of the first aspect, wherein the aerosol outlet channel is configured to receive aerosol generated in the aerosol provision system.

According to a third aspect of certain embodiments there is provided a method of controlling the temperature of a portion of a mouthpiece from an aerosol provision system, wherein the method comprises: operating a thermal element from the mouthpiece to control the temperature of the portion of the mouthpiece, wherein the portion comprises an exterior surface of the mouthpiece, wherein the thermal element comprises at least one of a heater and/or cooler.

It will be appreciated that features and aspects of the invention described above in relation to the various aspects of the invention are equally applicable to, and may be combined with, embodiments of the invention according to other aspects of the invention as appropriate, and not just in the specific combinations described herein.

### BRIEF DESCRIPTION OF THE DRAWINGS

Embodiments of the disclosure will now be described, by way of example only, with reference to the accompanying drawings, in which:
Figure 1 schematically represents in perspective view an aerosol provision system comprising a cartridge and aerosol provision device (shown separated) in accordance with certain embodiments of the disclosure.
Figure 2 schematically represents in exploded perspective view of components of the cartridge of the aerosol provision system of Figure 1.
Figures 3A to 3C schematically represent various cross-section views of a housing part of the cartridge of the aerosol provision system of Figure 1.
Figures 4A and 4B schematically represent a perspective view and a plan view of a dividing wall element of the cartridge of the aerosol provision system of Figure 1.
Figures 5A to 5C schematically represent two perspective views and a plan view of a resilient plug of the cartridge of the aerosol provision system of Figure 1.
Figures 6A and 6B schematically represent a perspective view and a plan view of a bottom cap of the cartridge of the aerosol provision system of Figure 1.
Figure 7 schematically represent a mouthpiece comprising a thermal element, which is useable with the aerosol provision systems described above with reference to Figures 1-6B, in accordance with certain embodiments of the disclosure.
Figure 8A schematically represents a type of thermal element which may be employable in the mouthpiece illustrated in Figure 7, in accordance with certain embodiments of the disclosure.
Figure 8B schematically represents a type of thermal element which may be employable in the mouthpiece illustrated in Figure 7, in accordance with certain embodiments of the disclosure.
Figure 9 schematically represent a mouthpiece comprising an annular thermal element, which is useable with the aerosol provision systems described above with reference to Figures 1-6B, in accordance with certain embodiments of the disclosure.
Figure 10 schematically represent an aerosol provision system employing a mouthpiece comprising a thermal element, such as the mouthpiece from Figures 7 and 9, in accordance with certain embodiments of the disclosure.

### DETAILED DESCRIPTION OF THE DRAWINGS

Aspects and features of certain examples and embodiments are discussed/described herein. Some aspects and features of certain examples and embodiments may be implemented conventionally and these are not discussed/described in detail in the interests of brevity. It will thus be appreciated that aspects and features of apparatus and methods discussed herein which are not described in detail may be implemented in accordance with any conventional techniques for implementing such aspects and features.

The present disclosure relates to non-combustible aerosol provision systems (such as an e-cigarette). According to the present disclosure, a "non-combustible" aerosol provision system is one where a constituent aerosolizable material of the aerosol provision system (or component thereof) is not combusted or burned in order to facilitate delivery to a user. Aerosolizable material, which also may be referred to herein as aerosol generating material or aerosol precursor material, is material that is capable of generating aerosol, for example when heated, irradiated or energized in any other way. The aerosolizable material may also be flavored, in some embodiments.

Throughout the following description the term "e-cigarette" or "electronic cigarette" may sometimes be used, but it will be appreciated this term may be used interchangeably with an aerosol provision system. An electronic cigarette may also be known as a vaping device or electronic nicotine delivery system (END), although it is noted that the presence of nicotine in the aerosolizable material is not a requirement.

In some embodiments, the aerosol provision system is a hybrid device configured to generate aerosol using a combination of aerosolizable materials, one or a plurality of which may be heated. In some embodiments, the hybrid device comprises a liquid or gel aerosolizable material and a solid aerosolizable material. The solid aerosolizable material may comprise, for example, tobacco or a non-tobacco product.

Typically, the (non-combustible) aerosol provision system may comprise a cartridge/consumable part and a body/reusable/aerosol provision device part, which is configured to releasably engage with the cartridge/consumable part.

The aerosol provision system may be provided with a means for powering a vaporizer therein, and there may be provided an aerosolizable material transport element for receiving the aerosolizable material that is to be vaporized. The aerosol provision system may also be provided with a reservoir for containing aerosolizable material, and in some embodiments a further reservoir for containing flavoring material for flavoring a generated vapor from the aerosol provision system.

In some embodiments, the vaporizer may be a heater/heating element capable of interacting with the aerosolizable material so as to release one or more volatiles from the aerosolizable material to form a vapor/aerosol. In some embodiments, the vaporizer is capable of generating an aerosol from the aerosolizable material without heating. For example, the vaporizer may be capable of generating a vapor/aerosol from the aerosolizable material without applying heat thereto, for example via one or more of vibrational, mechanical, pressurization or electrostatic means.

In some embodiments, the substance to be delivered may be an aerosolizable material which may comprise an active constituent, a carrier constituent and optionally one or more other functional constituents.

The active constituent may comprise one or more physiologically and/or olfactory active constituents which are included in the aerosolizable material in order to achieve a physiological and/or olfactory response in the user. The active constituent may for example be selected from nutraceuticals, nootropics, and psychoactives. The active constituent may be naturally occurring or synthetically obtained. The active constituent may comprise for example nicotine, caffeine, taurine, theine, a vitamin such as B6 or B12 or C, melatonin, a cannabinoid, or a constituent, derivative, or combinations thereof. The active constituent may comprise a constituent, derivative or extract of tobacco or of another botanical. In some embodiments, the active constituent is a physiologically active constituent and may be selected from nicotine, nicotine salts (e.g. nicotine ditartrate/nicotine bitartrate), nicotine-free tobacco substitutes, other alkaloids such as caffeine, or mixtures thereof.

In some embodiments, the active constituent is an olfactory active constituent and may be selected from a "flavor" and/or "flavorant" which, where local regulations permit, may be used to create a desired taste, aroma or other somatosensorial sensation in a product for adult consumers. In some instances such constituents may be referred to as flavors, flavorants, flavoring material, cooling agents, heating agents, and/or sweetening agents. They may include naturally occurring flavor materials, botanicals, extracts of botanicals, synthetically obtained materials, or combinations thereof (e.g., tobacco, cannabis, licorice (liquorice), hydrangea, eugenol, Japanese white bark magnolia leaf, chamomile, fenugreek, clove, maple, matcha, menthol, Japanese mint, aniseed (anise), cinnamon, turmeric, Indian spices, Asian spices, herb, wintergreen, cherry, berry, red berry, cranberry, peach, apple, orange, mango, clementine, lemon, lime, tropical fruit, papaya, rhubarb, grape, durian, dragon fruit, cucumber, blueberry, mulberry, citrus fruits, Drambuie, bourbon, scotch, whiskey, gin, tequila, rum, spearmint, peppermint, lavender, aloe vera, cardamom, celery, cascarilla, nutmeg, sandalwood, bergamot, geranium, khat, naswar, betel, shisha, pine, honey essence, rose oil, vanilla, lemon oil, orange oil, orange blossom, cherry blossom, cassia, caraway, cognac, jasmine, ylang-ylang, sage, fennel, wasabi, piment, ginger, coriander, coffee, hemp, a mint oil from any species of the genus Mentha, eucalyptus, star anise, cocoa, lemongrass, rooibos, flax, ginkgo biloba, hazel, hibiscus, laurel, mate, orange skin, rose, tea such as green tea or black tea, thyme, juniper, elderflower, basil, bay leaves, cumin, oregano, paprika, rosemary, saffron, lemon peel, mint, beefsteak plant, curcuma, cilantro, myrtle, cassis, valerian, pimento, mace, damien, marjoram, olive, lemon balm, lemon basil, chive, carvi, verbena, tarragon, limonene, thymol, camphene), flavor enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, liquid such as an oil, solid such as a powder, or gasone or more of extracts (e.g., licorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavor enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

In some embodiments, the flavoring material (flavor) may comprise menthol, spearmint and/or peppermint. In some embodiments, the flavor comprises flavor components of cucumber, blueberry, citrus fruits and/or redberry. In some embodiments, the flavor comprises eugenol. In some embodiments, the flavor comprises flavor components extracted from tobacco. In some embodiments, the flavor may comprise a sensate, which is intended to achieve a somatosensorial sensation which are usually chemically induced and perceived by the stimulation of the fifth cranial nerve (trigeminal nerve), in addition to or in place of aroma or taste nerves, and these may include agents providing heating, cooling, tingling, numbing effect. A suitable heat effect agent may be, but is not limited to, vanillyl ethyl ether and a suitable cooling agent may be, but not limited to eucalyptol, WS-3.

The carrier constituent may comprise one or more constituents capable of forming an aerosol. In some embodiments, the carrier constituent may comprise one or more of glycerine, glycerol, propylene glycol, diethylene glycol, triethylene glycol, tetraethylene glycol, 1,3-butylene glycol, erythritol, meso-Erythritol, ethyl vanillate, ethyl laurate, a diethyl suberate, triethyl citrate, triacetin, a diacetin mixture, benzyl benzoate, benzyl phenyl acetate, tributyrin, lauryl acetate, lauric acid, myristic acid, and propylene carbonate.

The one or more other functional constituents may comprise one or more of pH regulators, coloring agents, preservatives, binders, fillers, stabilizers, and/or antioxidants.

As noted above, aerosol provision systems (e-cigarettes) may often comprise a modular assembly including both a reusable part (body - or aerosol provision device) and a replaceable consumable (cartridge) part. Devices conforming to this type of two-part modular configuration may generally be referred to as two-part devices. It is also common for electronic cigarettes to have a generally elongate shape. For the sake of providing a concrete example, certain embodiments of the disclosure described herein may comprise this kind of generally elongate two-part device employing consumable parts. However, it will be appreciated the underlying principles described herein may equally be adopted for other electronic cigarette configurations, for example modular devices comprising more than two parts, as devices conforming to other overall shapes, for example based on so-called box-mod high performance devices that typically have a more boxy shape.

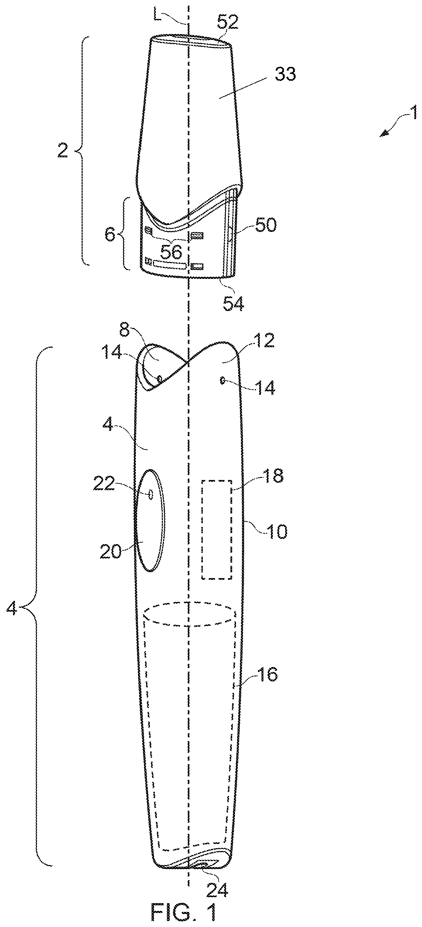

From the forgoing therefore, and with reference to Figure 1 is a schematic perspective view of an example aerosol provision system (e-cigarette) 1 in accordance with certain embodiments of the disclosure. Terms concerning the relative location of various aspects of the electronic cigarette (e.g. terms such as upper, lower, above, below, top, bottom etc.) are used herein with reference to the orientation of the electronic cigarette as shown in Figure 1 (unless the context indicates otherwise). However, it will be appreciated this is purely for ease of explanation and is not intended to indicate there is any required orientation for the electronic cigarette in use.

The e-cigarette 1 (aerosol provision system 1) comprises two main components, namely a cartridge 2 and an aerosol provision device 4. The aerosol provision device 4 and the cartridge 2 are shown separated in Figure 1, but are coupled together when in use.

The cartridge 2 and aerosol provision device 4 are coupled by establishing a mechanical and electrical connection between them. The specific manner in which the mechanical and electrical connection is established is not of primary significance to the principles described herein and may be established in accordance with conventional techniques, for example based around a screw thread, bayonet, latched or friction-fit mechanical fixing with appropriately arranged electrical contacts/electrodes for establishing the electrical connection between the two parts as appropriate. For example electronic cigarette 1 represented in Figure 1, the cartridge comprises a mouthpiece 33, a mouthpiece end 52 and an interface end 54 and is coupled to the aerosol provision device by inserting an interface end portion 6 at the interface end of the cartridge into a corresponding receptacle 8/receiving section of the aerosol provision device. The interface end portion 6 of the cartridge is a close fit to be receptacle 8 and includes protrusions 56 which engage with corresponding detents in the interior surface of a receptacle wall 12 defining the receptacle 8 to provide a releasable mechanical engagement between the cartridge and the aerosol provision device. An electrical connection is established between the aerosol provision device and the cartridge via a pair of electrical contacts on the bottom of the cartridge (not shown in Figure 1) and corresponding sprung contact pins in the base of the receptacle 8 (not shown in Figure 1). As noted above, the specific manner in which the electrical connection is established is not significant to the principles described herein, and indeed some implementations might not have an electrical connection between the cartridge and a aerosol provision device at all, for example because the transfer of electrical power from the reusable part to the cartridge may be wireless (e.g. based on electromagnetic induction techniques).

The electronic cigarette 1 (aerosol provision system) has a generally elongate shape extending along a longitudinal axis L. When the cartridge is coupled to the aerosol provision device, the overall length of the electronic cigarette in this example (along the longitudinal axis) is around 12.5 cm. The overall length of the aerosol provision device is around 9 cm and the overall length of the cartridge is around 5 cm (i.e. there is around 1.5 cm of overlap between the interface end portion 6 of the cartridge and the receptacle 8 of the aerosol provision device when they are coupled together). The electronic cigarette has a cross-section which is generally oval and which is largest around the middle of the electronic cigarette and tapers in a curved manner towards the ends. The cross-section around the middle of the electronic cigarette has a width of around 2.5 cm and a thickness of around 1.7 cm. The end of the cartridge has a width of around 2 cm and a thickness of around 0.6 mm, whereas the other end of the electronic cigarette has a width of around 2 cm and a thickness of around 1.2 cm. The outer housing of the electronic cigarette is in this example is formed from plastic. It will be appreciated the specific size and shape of the electronic cigarette and the material from which it is made is not of primary significance to the principles described herein and may be different in different implementations. That is to say, the principles described herein may equally be adopted for electronic cigarettes having different sizes, shapes and/or materials.

The aerosol provision device 4 may in accordance with certain embodiments of the disclosure be broadly conventional in terms of its functionality and general construction techniques. In the example of Figure 1, the aerosol provision device 4 comprises a plastic outer housing 10 including the receptacle wall 12 that defines the receptacle 8 for receiving the end of the cartridge as noted above. The outer housing 10 of the aerosol provision device 4 in this example has a generally oval cross section conforming to the shape and size of the cartridge 2 at their interface to provide a smooth transition between the two parts. The receptacle 8 and the end portion 6 of the cartridge 2 are symmetric when rotated through 180° so the cartridge can be inserted into the aerosol provision device in two different orientations. The receptacle wall 12 includes two aerosol provision device air inlet openings 14 (i.e. holes in the wall). These openings 14 are positioned to align with an air inlet 50 for the cartridge when the cartridge is coupled to the aerosol provision device. A different one of the openings 14 aligns with the air inlet 50 of the cartridge in the different orientations. It will be appreciated some implementations may not have any degree of rotational symmetry such that the cartridge is couplable to the aerosol provision device in only one orientation while other implementations may have a higher degree of rotational symmetry such that the cartridge is couplable to the aerosol provision device in more orientations.

The aerosol provision device further comprises a battery 16 for providing operating power for the electronic cigarette, control circuitry 18 for controlling and monitoring the operation of the electronic cigarette, a user input button 20, an indicator light 22, and a charging port 24.

The battery 16 in this example is rechargeable and may be of a conventional type, for example of the kind normally used in electronic cigarettes and other applications requiring provision of relatively high currents over relatively short periods. The battery 16 may be recharged through the charging port 24, which may, for example, comprise a USB connector.

The input button 20 in this example is a conventional mechanical button, for example comprising a sprung mounted component which may be pressed by a user to establish an electrical contact in underlying circuitry. In this regard, the input button may be considered an input device for detecting user input, e.g. to trigger aerosol generation, and the specific manner in which the button is implemented is not significant. For example, other forms of mechanical button or touch-sensitive button (e.g. based on capacitive or optical sensing techniques) may be used in other implementations, or there may be no button and the device may rely on a puff detector for triggering aerosol generation.

The indicator light 22 is provided to give a user with a visual indication of various characteristics associated with the electronic cigarette, for example, an indication of an operating state (e.g. on/off/standby), and other characteristics, such as battery life or fault conditions. Different characteristics may, for example, be indicated through different colors and/or different flash sequences in accordance with generally conventional techniques.

The control circuitry 18 is suitably configured/programmed to control the operation of the electronic cigarette to provide conventional operating functions in line with the established techniques for controlling electronic cigarettes. The control circuitry (processor circuitry) 18 may be considered to logically comprise various sub-units/circuitry elements associated with different aspects of the electronic cigarette's operation. For example, depending on the functionality provided in different implementations, the control circuitry 18 may comprises power supply control circuitry for controlling the supply of power from the battery/power supply to the cartridge in response to user input, user programming circuitry for establishing configuration settings (e.g. user-defined power settings) in response to user input, as well as other functional units / circuitry associated functionality in accordance with the principles described herein and conventional operating aspects of electronic cigarettes, such as indicator light display driving circuitry and user input detection circuitry. It will be appreciated the functionality of the control circuitry 18 can be provided in various different ways, for example using one or more suitably programmed programmable computer(s) and/or one or more suitably configured application-specific integrated circuit(s)/circuitry/chip(s)/chipset(s) configured to provide the desired functionality.

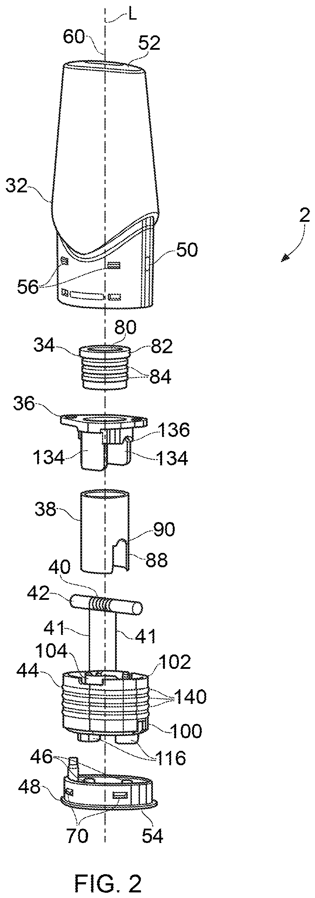

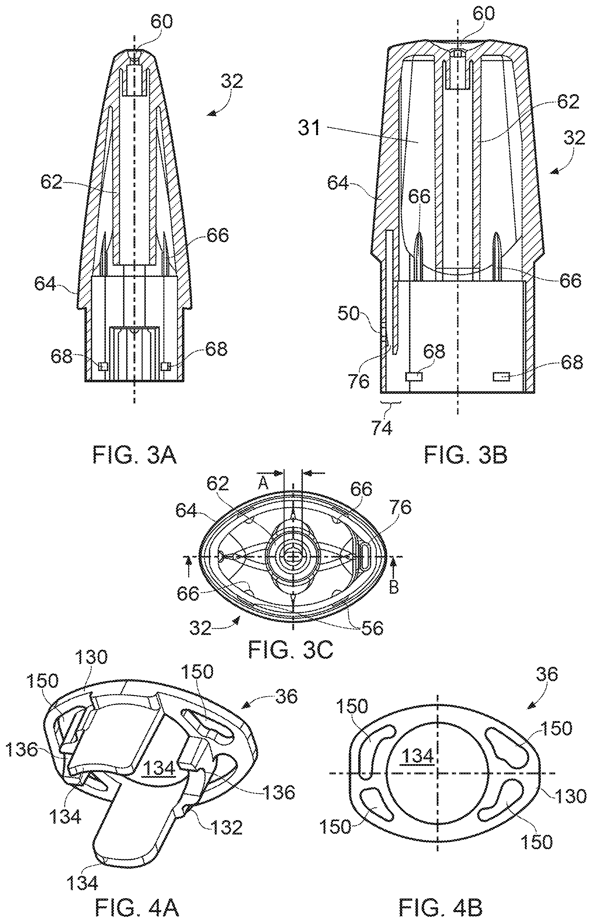

Figure 2 is an exploded schematic perspective view of the cartridge 2 (exploded along the longitudinal axis L). The cartridge 2 comprises a housing part 32, an air channel seal 34, a dividing wall element 36, an outlet tube 38, a vaporizer/heating element 40, an aerosolizable material transport element 42, a plug 44, and an end cap 48 with contact electrodes 46. Figures 3 to 6 schematically represents some of these components in more detail.

Figure 3A is a schematic cut-away view of the housing part 32 through the longitudinal axis L where the housing part 32 is thinnest. Figure 3B is a schematic cut-away view of the housing part 32 through the longitudinal axis L where the housing part 32 is widest. Figure 3C is a schematic view of the housing part along the longitudinal axis L from the interface end 54 (i.e. viewed from below in the orientation of Figures 3A and 3B).

Figures 4A is a schematic perspective view of the dividing wall element 36 as seen from below. Figure 4B is a schematic cross-section through an upper part of the dividing wall element 36 as viewed from below.

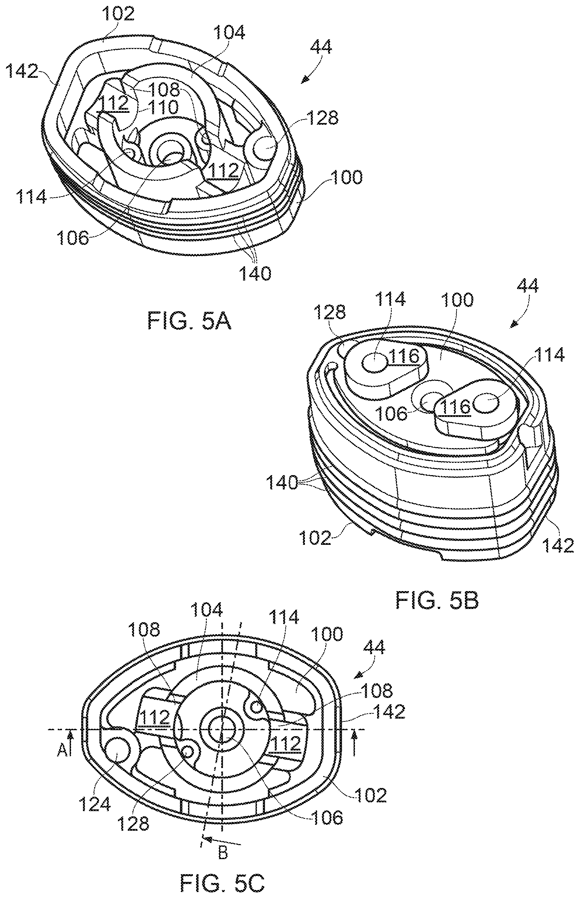

Figure 5A is a schematic perspective view of the plug 44 from above and Figure 5B is a schematic perspective view of the plug 44 from below. Figure 5C is a schematic view of the plug 44 along the longitudinal axis L seen from the mouthpiece end 52 of the cartridge (i.e. viewed from above for the orientation in Figures 1 and 2).

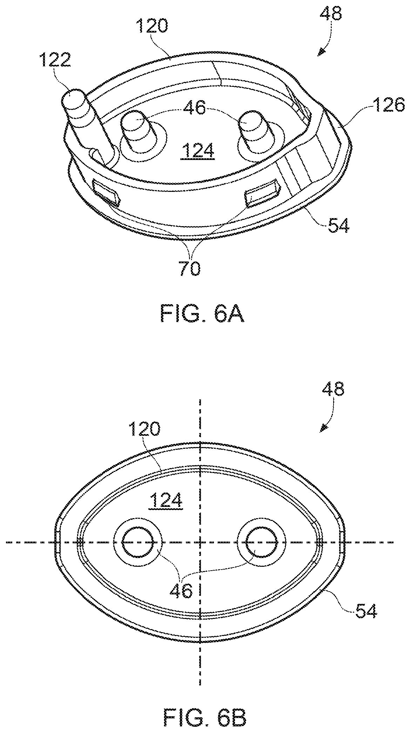

Figure 6A is a schematic perspective view of the end cap 48 from above. Figure 6B is a schematic view of the end cap 48 along the longitudinal axis L seen from the mouthpiece end 52 of the cartridge (i.e. from above).

The housing part 32 in this example comprises a housing outer wall 64 and a housing inner tube 62 which in this example are formed from a single molding of polypropylene. The housing outer wall 64 defines the external appearance of the cartridge 2 and the housing inner tube 62 defines a part the air channel through the cartridge. The housing part is open at the interface end 54 of the cartridge and closed at the mouthpiece end 52 of the cartridge except for a mouthpiece opening/aerosol outlet 60, from the mouthpiece 33, which is in fluid communication with the housing inner tube 62. The housing part 32 includes an opening in a sidewall which provides the air inlet 50 for the cartridge. The air inlet 50 in this example has an area of around 2 mm². The outer surface of the outer wall 64 of the housing part 32 includes the protrusions 56 discussed above which engage with corresponding detents in the interior surface of the receptacle wall 12 defining the receptacle 8 to provide a releasable mechanical engagement between the cartridge and the aerosol provision device. The inner surface of the outer wall 64 of the housing part includes further protrusions 66 which act to provide an abutment stop for locating the dividing wall element 36 along the longitudinal axis L when the cartridge is assembled. The outer wall 64 of the housing part 32 further comprises holes which provide latch recesses 68 arranged to receive corresponding latch projections 70 in the end cap to fix the end cap to be housing part when the cartridge is assembled.

The outer wall 64 of the housing part 32 includes a double-walled section 74 that defines a gap 76 in fluid communication with the air inlet 50. The gap 76 provides a portion of the air channel through the cartridge. In this example the doubled-walled section 74 of the housing part 32 is arranged so the gap defines an air channel running within the housing outer wall 64 parallel to the longitudinal axis with a cross-section in a plane perpendicular to the longitudinal axis of around 3 mm². The gap/portion of air channel 76 defined by the double-walled section of the housing part extends down to the open end of the housing part 32.

The air channel seal 34 is a silicone molding generally in the form of a tube having a through hole 80. The outer wall of the air channel seal 34 includes circumferential ridges 84 and an upper collar 82. The inner wall of the air channel seal 34 also includes circumferential ridges, but these are not visible in Figure 2. When the cartridge is assembled the air channel seal 34 is mounted to the housing inner tube 62 with an end of the housing inner tube 62 extending partly into the through hole 80 of the air channel seal 34. The through hole 80 in the air channel seal has a diameter of around 5.8 mm in its relaxed state whereas the end of the housing inner tube 62 has a diameter of around 6.2 mm so that a seal is formed when the air channel seal 34 is stretched to accommodate the housing inner tube 62. This seal is facilitated by the ridges on the inner surface of the air channel seal 34.

The outlet tube 38 comprises a tubular section, for instance made of ANSI 304 stainless steel or polypropylene, with an internal diameter of around 8.6 mm and a wall thickness of around 0.2 mm. The bottom end of the outlet tube 38 includes a pair of diametrically opposing slots 88 with an end of each slot having a semi-circular recess 90. When the cartridge is assembled the outlet tube 38 mounts to the outer surface of the air channel seal 34. The outer diameter of the air channel seal is around 9.0 mm in its relaxed state so that a seal is formed when the air channel seal 34 is compressed to fit inside the outlet tube 38. This seal is facilitated by the ridges 84 on the outer surface of the air channel seal 34. The collar 80 on the air channel seal 34 provides a stop for the outlet tube 38.

The aerosolizable material transport element 42 comprises a capillary wick and the vaporizer (aerosol generator) 40 comprises a resistance wire heater wound around the capillary wick. In addition to the portion of the resistance wire wound around the capillary wick, the vaporizer comprises electrical leads 41 which pass through holes in the plug 44 to contact electrodes 46 mounted to the end cap 54 to allow power to be supplied to the vaporizer via the electrical interface the established when the cartridge is connected to an aerosol provision device. The vaporizer leads 41 may comprise the same material as the resistance wire wound around the capillary wick, or may comprise a different material (e.g. lower-resistance material) connected to the resistance wire wound around the capillary wick. In this example the heater coil 40 comprises a nickel iron alloy wire and the wick 42 comprises a glass fiber bundle. The vaporizer and aerosolizable material transport element may be provided in accordance with any conventional techniques and is may comprise different forms and/or different materials. For example, in some implementations the wick may comprise fibrous or solid a ceramic material and the heater may comprise a different alloy. In other examples the heater and wick may be combined, for example in the form of a porous and a resistive material. More generally, it will be appreciated the specific nature aerosolizable material transport element and vaporizer is not of primary significance to the principles described herein.

When the cartridge is assembled, the wick 42 is received in the semi-circular recesses 90 of the outlet tube 38 so that a central portion of the wick about which the heating coil is would is inside the outlet tube while end portions of the wick are outside the outlet tube 38.

The plug 44 in this example comprises a single molding of silicone, may be resilient. The plug comprises a base part 100 with an outer wall 102 extending upwardly therefrom (i.e. towards the mouthpiece end of the cartridge). The plug further comprises an inner wall 104 extending upwardly from the base part 100 and surrounding a through hole 106 through the base part 100.

The outer wall 102 of the plug 44 conforms to an inner surface of the housing part 32 so that when the cartridge is assembled the plug in 44 forms a seal with the housing part 32. The inner wall 104 of the plug 44 conforms to an inner surface of the outlet tube 38 so that when the cartridge is assembled the plug 44 also forms a seal with the outlet tube 38. The inner wall 104 includes a pair of diametrically opposing slots 108 with the end of each slot having a semi-circular recess 110. Extended outwardly (i.e. in a direction away from the longitudinal axis of the cartridge) from the bottom of each slot in the inner wall 104 is a cradle section 112 shaped to receive a section of the aerosolizable material transport element 42 when the cartridge is assembled. The slots 108 and semi-circular recesses 110 provided by the inner wall of the plug 44 and the slots 88 and semi-circular recesses 90 of the outlet tube 38 are aligned so that the slots 88 in the outlet tube 38 accommodate respective ones of the cradles 112 with the respective semi-circular recesses in the outlet tube and plug cooperating to define holes through which the aerosolizable material transport element passes. The size of the holes provided by the semi-circular recesses through which the aerosolizable material transport element passes correspond closely to the size and shape of the aerosolizable material transport element, but are slightly smaller so a degree of compression is provided by the resilience of the plug 44. This allows aerosolizable material to be transported along the aerosolizable material transport element by capillary action while restricting the extent to which aerosolizable material which is not transported by capillary action can pass through the openings. As noted above, the plug 44 includes further openings 114 in the base part 100 through which the contact leads 41 for the vaporizer pass when the cartridge is assembled. The bottom of the base part of the plug includes spacers 116 which maintain an offset between the remaining surface of the bottom of the base part and the end cap 48. These spacers 116 include the openings 114 through which the electrical contact leads 41 for the vaporizer pass.

The end cap 48 comprises a polypropylene molding with a pair of gold-plated copper electrode posts 46 mounted therein.

The ends of the electrode posts 44 on the bottom side of the end cap are close to flush with the interface end 54 of the cartridge provided by the end cap 48. These are the parts of the electrodes to which correspondingly aligned sprung contacts in the aerosol provision device 4 connect when the cartridge 2 is assembled and connected to the aerosol provision device 4. The ends of the electrode posts on the inside of the cartridge extend away from the end cap 48 and into the holes 114 in the plug 44 through which the contact leads 41 pass. The electrode posts are slightly oversized relative to the holes 114 and include a chamfer at their upper ends to facilitate insertion into the holes 114 in the plug where they are maintained in pressed contact with the contact leads for the vaporizer by virtue of the plug.

The end cap has a base section 124 and an upstanding wall 120 which conforms to the inner surface of the housing part 32. The upstanding wall 120 of the end cap 48 is inserted into the housing part 32 so the latch projections 70 engage with the latch recesses 68 in the housing part 32 to snap-fit the end cap 48 to the housing part when the cartridge is assembled. The top of the upstanding wall 120 of the end cap 48 abuts a peripheral part of the plug 44 and the lower face of the spacers 116 on the plug also abut the base section 124 of the plug so that when the end cap 48 is attached to the housing part it presses against the resilient part 44 to maintain it in slight compression.

The base portion 124 of the end cap 48 includes a peripheral lip 126 beyond the base of the upstanding wall 112 with a thickness which corresponds with the thickness of the outer wall of the housing part at the interface end of the cartridge. The end cap also includes an upstanding locating pin 122 which aligns with a corresponding locating hole 128 in the plug to help establish their relative location during assembly.

The dividing wall element 36 comprises a single molding of polypropylene and includes a dividing wall 130 and a collar 132 formed by projections from the dividing wall 130 in the direction towards the interface end of the cartridge. The dividing wall element 36 has a central opening 134 through which the outlet tube 38 passes (i.e. the dividing wall is arranged around the outlet tube 38). **In** some embodiments, the dividing wall element 36 may be integrally formed with the outlet tube 38. When the cartridge is assembled, the upper surface of the outer wall 102 of the plug 44 engages with the lower surface of the dividing wall 130, and the upper surface of the dividing wall 130 in turn engages with the projections 66 on the inner surface of the outer wall 64 of the housing part 32. Thus, the dividing wall 130 prevents the plug from being pushed too far into the housing part 32 - i.e. the dividing wall 130 is fixedly located along the longitudinal axis of the cartridge by the protrusions 66 in the housing part and so provides the plug with a fixed surface to push against. The collar 132 formed by projections from the dividing wall includes a first pair of opposing projections/tongues 134 which engage with corresponding recesses on an inner surface of the outer wall 102 of the plug 44. The protrusions from the dividing wall 130 further provide a pair of cradle sections 136 configured to engage with corresponding ones of the cradle sections 112 in the part 44 when the cartridge is assembled to further define the opening through which the aerosolizable material transport element passes.

When the cartridge 2 is assembled an air channel extending from the air inlet 50 to the aerosol outlet 60 through the cartridge is formed. Starting from the air inlet 50 in the side wall of the housing part 32, a first section of the air channel is provided by the gap 76 formed by the double-walled section 74 in the outer wall 64 of the housing part 32 and extends from the air inlet 50 towards the interface end 54 of the cartridge and past the plug 44. A second portion of the air channel is provided by the gap between the base of the plug 44 and the end cap 48. A third portion of the air channel is provided by the hole 106 through the plug 44. A fourth portion of the air channel is provided by the region within the inner wall 104 of the plug and the outlet tube around the vaporizer 40. This fourth portion of the air channel may also be referred to as an aerosol/aerosol generation region, it being the primary region in which aerosol is generated during use. The air channel from the air inlet 50 to the aerosol generation region may be referred to as an air inlet section of the air channel. A fifth portion of the air channel is provided by the remainder of the outlet tube 38. A sixth portion of the air channel is provided by the outer housing inner tube 62 which connects the air channel to the aerosol outlet 60, which is located at an end of the mouthpiece 33. The air channel from the aerosol generation region to be the aerosol outlet may be referred to as an aerosol outlet section of the air channel.

Also, when the cartridge is assembled a reservoir 31 for aerosolizable material is formed by the space outside the air channel and inside the housing part 32. This may be filled during manufacture, for example through a filling hole which is then sealed, or by other means. The specific nature of the aerosolizable material, for example in terms of its composition, is not of primary significance to the principles described herein, and in general any conventional aerosolizable material of the type normally used in electronic cigarettes may be used. The present disclosure may refer to a liquid as the aerosolizable material, which as mentioned above may be a conventional e-liquid. However, the principles of the present disclosure apply to any aerosolizable material which has the ability to flow, and may include a liquid, a gel, or a solid, where for a solid a plurality of solid particles may be considered to have the ability to flow when considered as a bulk.

The reservoir is closed at the interface end of the cartridge by the plug 44. The reservoir includes a first region above the dividing wall 130 and a second region below the dividing wall 130 within the space formed between the air channel and the outer wall of the plug. The aerosolizable material transport element (capillary wick) 42 passes through openings in the wall of the air channel provided by the semi-circular recesses 108, 90 in the plug 44 and the outlet tube 38 and the cradle sections 112, 136 in the plug 44 and the dividing wall element 36 that engage with one another as discussed above. Thus, the ends of the aerosolizable material transport element extend into the second region of the reservoir from which they draw aerosolizable material through the openings in the air channel to the vaporizer 40 for subsequent vaporization.

In normal use, the cartridge 2 is coupled to the aerosol provision device 4 and the aerosol provision device activated to supply power to the cartridge via the contact electrodes 46 in the end cap 48. Power then passes through the connection leads 41 to the vaporizer 40. The vaporizer is thus electrically heated and so vaporizes a portion of the aerosolizable material from the aerosolizable material transport element in the vicinity of the vaporizer. This generates aerosol in the aerosol generation region of the air path. Aerosolizable material that is vaporized from the aerosolizable material transport element is replaced by more aerosolizable material drawn from the reservoir by capillary action. While the vaporizer is activated, a user inhales on the mouthpiece end 52 of the cartridge. This causes air to be drawn through whichever aerosol provision device air inlet 14 aligns with the air inlet 50 of the cartridge (which will depend on the orientation in which the cartridge was inserted into the aerosol provision device receptacle 8). Air then enters the cartridge through the air inlet 50, passes along the gap 76 in the double-walled section 74 of the housing part 32, passes between the plug 44 and the end cap 48 before entering the aerosol generation region surrounding the vaporizer 40 through the hole 106 in the base part 100 of the plug 44. The incoming air mixes with aerosol generated from the vaporizer to form a condensation aerosol, which is then drawn along the outlet tube 38 and the housing part inner 62 before exiting through the mouthpiece outlet/aerosol outlet 60 for user inhalation.

From the above Figures 1-6B, it can be seen a possible embodiment construction of aerosol provision system 1 which is configured for generating an aerosol, which is suitable for use in the context of the present disclosure (alongside potentially other forms of aerosol provision system).

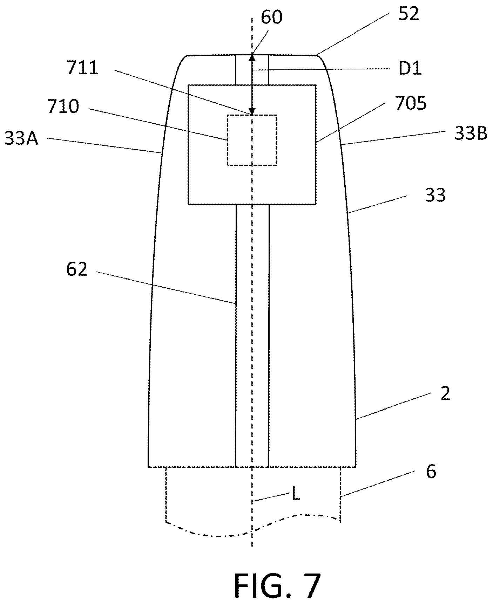

Turning now to Figures 7-10, the present disclosure also provides for a mouthpiece 33, which may be useable with any of the cartridges 2, aerosol provision devices 4, or aerosol provision systems herein described, such as any of those from the embodiments of Figures 1-6B for instance. At a general level therefore, the mouthpiece 33 may comprise a thermal element 710, such as a heater and/or cooler, for controlling the temperature of a portion 705 of the mouthpiece 33. In this way, and at a general level, the thermal element 710 may be configured to provide thermal feedback to a user of the mouthpiece 33. In this way, and upon an operation of the aerosol provision system when the portion 705 of the mouthpiece 33 is contacting the user's lips/mouth, the user may discern an operation or event of the aerosol provision system via appropriate thermal feedback delivered by the thermal element 710 to the portion 705 of the mouthpiece 33 at the appropriate time. Appreciating the above therefore, it may be seen that the provision of the thermal element 710 may allow for the improvement in user experience when using the aerosol provision system 300 by changing the temperature of the portion 705 of the mouthpiece 33 according to a number of different factors as explained in more detail below. Thus, the introduction of a thermal element 710 in the mouthpiece 33 may help to increase the operability/usability of the aerosol provision system 300.

According to the present disclosure, the thermal element 710 in accordance with some embodiments may be configured to either heat, cool, or a combination of both.

Where the thermal element 710 is configured for heating, the thermal element 710 in accordance with some embodiments thereof may comprise an electrical resistor located at the portion of the mouthpiece 33 for heating the portion 705 of the mouthpiece 33. This arrangement benefits from a simple implementation arising from the compact nature of electrical resistors, as well as the ease of access to such components. Electrical resistors are also easily controlled by an aerosol provision device4 / control circuitry 18 to apply a desired amount of thermal energy by modulating the voltage or current passing through the resistor, or alternatively by modulating the time that an electric current passes through the resistor.

The electrical resistor can take the form of an electrical coil in some embodiments according to the disclosure. This form provides the benefit of allowing heating of an increased surface area and offers greater flexibility in which parts of the mouthpiece 33 are heated.

Alternatively, in some embodiments the electrical resistor might take the form of a mesh shaped component located at the portion 705 of the mouthpiece 33. This form provides the benefit of allowing the thermal element 710 to more closely match the contours of the portion 705 of the mouthpiece 33, such that more uniform heating may be achieved.

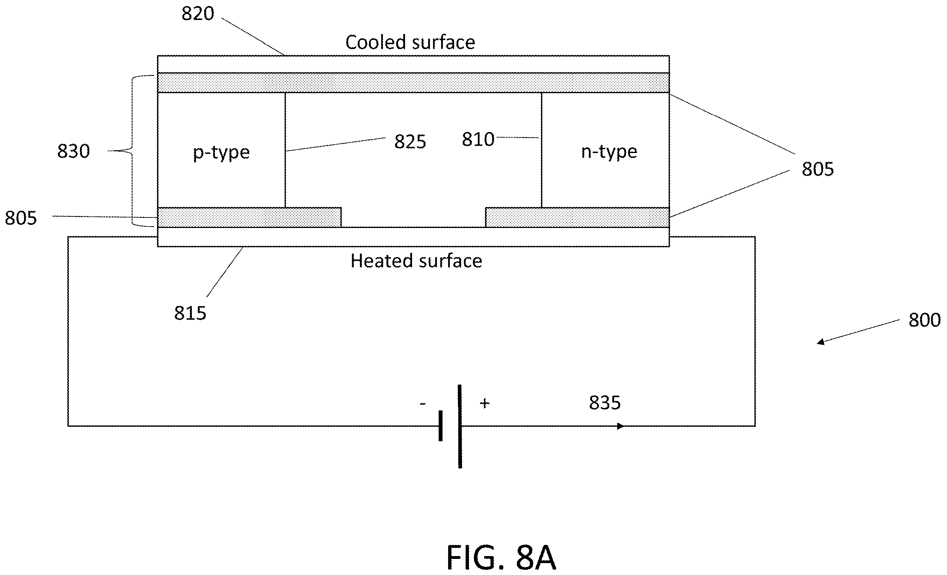

As mentioned above, the thermal element 710 may alternatively or additionally be configured for cooling. In a very particular embodiment for instance, a cooling effect might be achieved by providing a Peltier module 800 in the mouthpiece 33. As illustrated in Figure 8A, a Peltier module utilizes the Peltier effect to create a heat flux at the junction of two different conductors: a p-type conductor 825 and an n-type conductor 810. The conductors 810 and 825 are connected together with conductive contacts 805 as illustrated in Figure 8A. The Peltier module is a solid-state active heat pump capable of transferring heat from one side of the module to the other by consuming electrical energy. As illustrated in Figure 8A, the Peltier module 800 comprises two surfaces (a "hot" side 815 and a "cold" side 820) separated by an interconnect layer which may comprise one or more conductor junctions 830 connected in series.

When a DC current 835 is applied in one direction through the Peltier module, a first surface 820 of the module is cooled (thus forming the "cold" side) and a second surface 815 of the module is heated (thus forming the "hot" side).

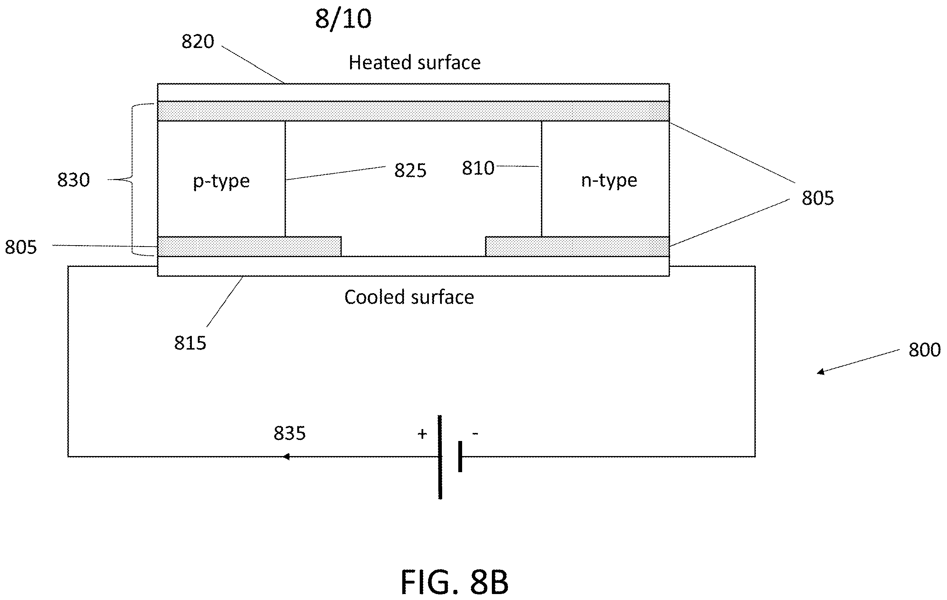

As illustrated in Figure 8B, when a DC current 835 is applied in the opposite direction to that in Figure 8A, the operation of the module is also reversed such that the first surface 820 is heated and the second surface 815 is cooled.

It will therefore be appreciated that a Peltier module 800 can advantageously be configured to provide both a heating and cooling effect to the portion 705 of the mouthpiece 33 when integrated into the mouthpiece 33.

Moving away from the operation of the thermal element 710, and turning to the location of the portion 705 on the mouthpiece 33, it will be appreciated that the portion 705 may be located anywhere on the mouthpiece 33 such that any heating or cooling thereof may be appropriately felt by the user of the mouthpiece 33 in use. This being the case, and according to some embodiments, the portion 705 of the mouthpiece 33 may conveniently comprise at least one external surface of the mouthpiece 33. This means that the user may make direct contact (e.g. with their lips and/or mouth, or with one or more hands) with the portion 705 acted upon by the thermal element 710 when using the aerosol provision system. Thus, thermal feedback can be provided to the user regarding the operation of the aerosol provision system 300 through the external surface of the mouthpiece 33. In this respect as well, for embodiments where the user contact is intended to be made via their lips, it is to be noted that in accordance with some embodiments, the presence of this external surface of the mouthpiece 33 may be also be a contoured surface, or a contoured recess, whose shape is configured to at least partially conform to the shape of a human lip, such to maximize the contact surface area between this external surface and the user's mouth/lip in use.

In other implementations, it should be appreciated that the portion 705 of the mouthpiece 33 may comprise a plurality of thermal elements 710. The plurality of thermal elements 710 may be activated together and provide effectively an increased heating area (i.e., a greater area of the external surface of the mouthpiece is heated/cooled). Alternatively or additionally, the plurality of thermal elements 710 may be controlled individually to provide any desired heating / cooling effect. For example, the plurality of heating elements 710 may be arranged and / or controlled to provide a thermal gradient across the external surface of the mouthpiece in one of more directions. The specific nature of the heating/cooling pattern may indicate further more complex feedback to the user.

With respect to the above embodiments, and potentially other embodiments as well, to allow for the effects of the thermal element 710 to be more easily felt by the mouth of the user when the portion 705 is in use, in accordance with some embodiments (such as that illustrated in the embodiment of Figure 7), the thermal element 710 may be located no more than 50mm from at least one of a mouthpiece opening (or aerosol outlet 60) from the mouthpiece 33, and/or from a mouthpiece end 52 of an aerosol provision system or cartridge 2 employing the mouthpiece 33. In accordance with some narrower embodiments, the thermal element 710 may be located no more than 45mm, no more than 40mm, no more than 35mm, no more than 30mm, no more than 25mm, and/or no more than 20mm, from at least one of the mouthpiece opening (or aerosol outlet 60) from the mouthpiece 33, and/or from the mouthpiece end 52. By the phrase 'no more than' here, this may be understood as meaning that at least one portion 711 of the thermal element 710 is no more than specified distance D1 away, rather than the entirety of the thermal element 710 necessarily needing to be no more than specified distance away.

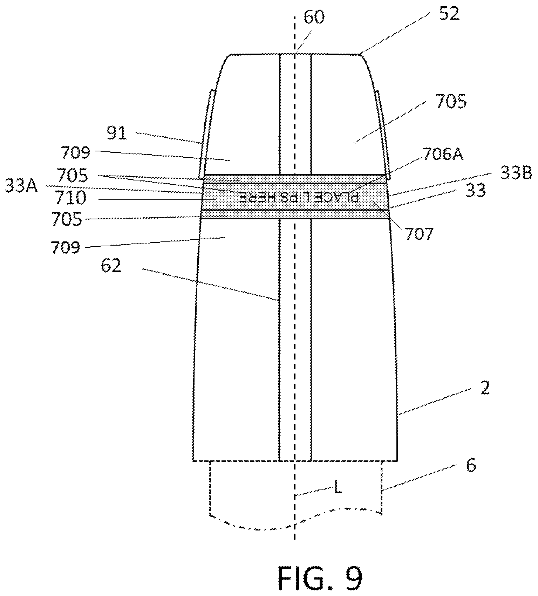

According to some embodiments, such as that shown in the embodiment of Figure 9, the portion 705 of the mouthpiece 33, and/or the thermal element 710, may surround, at least partially or completely, any provided aerosol outlet channel 62 (such as the tube 62 shown in the embodiments relating to Figures 1-6B and 7-10) or mouthpiece end 52 of the mouthpiece 33. In this way, the portion of the mouthpiece 33 may be positioned in such a way that the portion 705 may be proximal different circumferential areas of the user's lips when the user is using the mouthpiece 33.

Equally, in accordance with some embodiments (such as that shown in the embodiment of Figure 9), the portion 705 may comprise a first external surface 33A on a first side of the mouthpiece 33, and/or comprise a second external surface 33B on a second side of the mouthpiece 33. In some particular embodiments thereof, the first side 33A may be opposite the first side 33B of the mouthpiece 33.

To facilitate the portion 705 being a portion which is particularly suited for heating a user's mouth area, the portion 705 (and/or including any first external surface 33A and/or second first external surface 33B therefrom) may comprise at least one surface of the mouthpiece 33 which is inclined towards an aerosol outlet 60 located at an end 52 of the mouthpiece 33, as shown in the embodiment of Figure 9. Thus in a very particular embodiment, the portion 705 may comprise the first external surface 33A on the first side of the mouthpiece 33, and comprise the second external surface 33B on the second side of the mouthpiece 33, wherein each of the first and second external surfaces 33A;33B are inclined towards the end 52 of the mouthpiece 33.

Any such end 52, i.e. the mouthpiece end 52 shown in the embodiment of Figure 2 for instance, may in accordance with some particular embodiments be located between the first external surface 33A and the second external surface 33B (as shown in the embodiment of Figure 9).

Equally, in accordance with some embodiments (such as that shown in the embodiment of Figure 9), the portion 705 may comprise an annular portion which extends around the aerosol outlet channel 62 and/or the mouthpiece end 52. In such embodiments therefore, the portion 705 may be located between the end 52 of the mouthpiece 33, and the interface end portion 6 from any cartridge 2 which comprises the mouthpiece 33, as shown in the embodiments from the Figures.

With respect to the geometry of the mouthpiece end 52, in some embodiments this may define a substantially flat surface which surrounds the aerosols outlet 60, as shown in the embodiment of Figure 9 for instance.

To assist any user of the mouthpiece in being able to identify the location of the portion 75 (e.g. at the point when the user may be approaching the mouthpiece to place their mouth over it), the mouthpiece in accordance with some embodiments may comprise at least one indicia for identifying the location of the portion 705. At to what each such indicia might be, it will be appreciated that this could be a variety of different things. For instance, in accordance with some embodiments, the at least one indicia may comprise a tactile indicia, such as a protuberance or recess. In this way, the indicia may in accordance with some embodiments either project from, or be recessed in, an external surface of the mouthpiece 33 as shown in the embodiment from Figure 9.

Equally, in accordance with some embodiments, the at least one indicia may comprise a visual indicia, such as a portion of text, or a graphical symbol, 706A. Appreciably, the indicia could comprise a combination of a tactile and a visual indicia in accordance with some embodiments (such as raised or recessed text, and/or a raised or recessed graphical symbol).

Additionally/alternatively, with respect to the at least one indicia, in accordance with some embodiments, the at least one indicia 706 may comprise the portion 705 being colored with a first color 707 (e.g. not black, such as grey, orange, red, or white, or even colorless) which is different from a color (e.g. black) of a second portion 709 of the mouthpiece 33 which surrounds the portion 705 of the mouthpiece 33.

Staying with any employed at least one indicia, it will also be appreciated that in accordance with some embodiments, the at least one indicia may comprise the portion 705 being made of a different material (e.g. a metal or metal alloy, such as copper or aluminum in some very particular embodiments) from the material (e.g. a plastic, such as polycarbonate or polypropylene in some very particular embodiments) of the second portion 709 of the mouthpiece 33 which surrounds the portion 705 of the mouthpiece 33. In accordance with some embodiments, the material of the portion 705 may be more thermally conductive than the material of the second portion 709, such to better contain any heating or cooling effects from the thermal element 710 to the vicinity of the portion 705.

Also with respect to the at least one indicia, to help allow the user to more easily interpret the indicia as they are about to use the mouthpiece, the indicia in accordance with some embodiments may be orientated towards the end 52 of the mouthpiece 33 (as illustrated in the embodiment of Figure 9, where the text 'PLACE LIPS HERE' is orientated towards the end 52 of the mouthpiece 33).

In accordance with yet further embodiments where the at least one indicia is employed, in accordance with some embodiments thereof, the portion 705 may define at least one recess, wherein the at least one indicia comprises the at least one recess. In this way, each recess may in some particular embodiments be configured for allowing a lip of a user of the mouthpiece to be at least partially accommodated in the recess. In this way therefore, by virtue of each such recess being employed, this may effectively allow the user to quickly locate their lips in a predetermined correction position about the mouthpiece 33, and about the portion 705, by placing their lip in the recess.

Moving away from any provide indicia for identifying the location of the portion 705, with respect to the aerosol outlet channel 62 of the mouthpiece 33, this according to some embodiments may be configured to be thermally insulated from the thermal element 710 and the portion 705 of the mouthpiece 33. This advantageously ensures that the heating or cooling effect of the thermal element 710 does not adversely affect the temperature of any aerosol inside the aerosol outlet channel 62. In other words, by thermally insulating the temperature change induced by the thermal element 710, the temperature of the aerosol inside the vapor outlet channel 62 is left unaffected. Such thermal insulation may be implemented by providing a layer of insulating material (e.g. polyurethane) between the thermal element 710 and the aerosol outlet channel 62, in a very particular embodiment, for instance. Alternatively, the thermal insulation may be better achieved through the thermal element 710 being located no closer than 4mm, no closer than 6mm, no closer than 8mm, and/or no closer than 10mm, from the vapor outlet channel 62.

In accordance with some embodiments, and as illustrated in Figure 9, the mouthpiece 33 may in some cases comprise a sensor, such as a pressure sensor 91, configured to generate sensor data for use in controlling the operation of the thermal element 710. In particular, the sensor may be configured to allow the activation of the thermal element 710 based on the sensor data being determined as meeting one or more predetermined criterion. In accordance with the embodiments disclosed above, operation of the sensor 91 may result in the thermal element 710 activating and consequentially either raising or lowering the temperature of the portion 705 of the mouthpiece 33 depending upon the configuration of the thermal element 710. Any such sensor, such as a pressure sensor 91, may appreciably be located in a number of different locations. For instance, in accordance with the embodiment from Figure 9, the sensor 91 may be located on an external surface of the mouthpiece 33, and/or may be annular such that any portion of the user's mouth may engage the sensor (which would be especially helpful where the sensor is configured to either act as pressure sensor for detecting the presence of the user's mouth, or a different type of sensor configured to determine a property of the user's mouth, e.g. a temperature thereof). In some very particular embodiments, the portion 705 may comprise the sensor 91.

Related to any provision of such a sensor 91, in accordance with some embodiments, the mouthpiece may additionally/alternatively comprise a switch 91, which may be lip-actuatable in accordance with some embodiments, for controlling an operation from the aerosol provision system. Such an operation could be a number of different things, for instance in accordance with some embodiments an operation of the aerosol generator 40, and/or an operation to control the power supplied to the aerosol generator 40. As shown in the embodiment shown from Figure 9, the switch may in accordance with some embodiments be located on the at least one external surface 33A;33B. Equally, in accordance with some embodiments, the portion may comprise the switch 91.

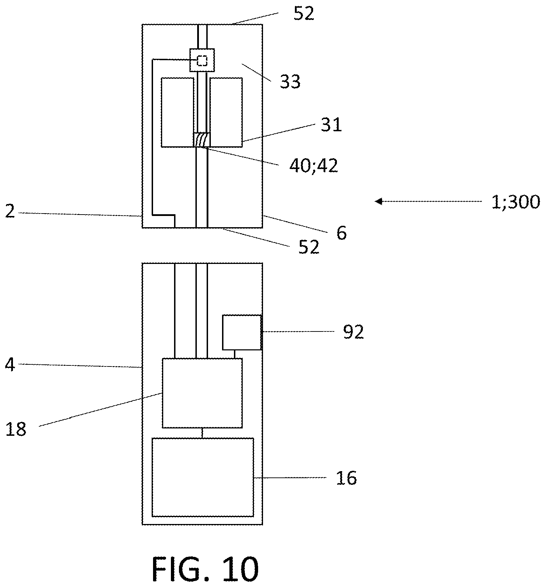

As illustrated in Figure 10, the mouthpiece 33 disclosed in the aforementioned embodiments may form part of an aerosol provision device or system for generating aerosol wherein the aerosol outlet channel 62 of the mouthpiece 33 is configured to receive aerosol generated in the aerosol provision system 300, such as the aerosol provision systems described with reference to the above Figures 1-6B in accordance with some embodiments. The aerosol provision system may comprise a cartridge 2 and an aerosol provision device 4 configured to receive the cartridge 2, wherein the cartridge 2 comprises the mouthpiece 33. Though in accordance with some embodiments, any provided aerosol provision device 4 may instead comprise the mouthpiece 33, and any such aerosol provision device 4 need not necessarily employ a cartridge 2.

According to some embodiments, the aerosol provision system may further comprise control circuitry 18 which is configured to: determine whether an output signal, for operating the thermal element, should be generated; and generate the output signal in response to determining that the output signal should be generated. In this way, the thermal element 710 is able to be selectively activated in response to a control signal generated by the control circuitry 18, which is a control signal which itself may be generated in response to a number of different stimuli as explained in greater detail below.

For instance, and according to some embodiments, the aerosol provision system may further comprise a sensor 91;92 for generating sensor data, wherein the control circuitry 18 is configured to: receive the sensor data from the sensor 91;92; and process the sensor data to determine whether the output signal should be generated. This provides a means for providing feedback to the user on the parameter being measured by the sensor 91;92 by way of activating the thermal element 710 based on a measurement of the parameter.

According to some embodiments, the sensor may comprise a temperature sensor 92, wherein the sensor data comprises a temperature value indicative of a temperature. For example, the temperature being sensed may be an ambient temperature of the environment surrounding the aerosol provision system 300. In this case, the thermal element 710 provides feedback to the user as to the ambient temperature. In other examples, the temperature being sensed may be related to a specific part of the aerosol provision system. For example, the temperature may be that of the aerosol passing through the aerosol outlet channel 62, a reservoir (such as the reservoir 31 shown in the embodiments of Figures 1-6B), or any other part of the aerosol provision system.

According to some embodiments, the output signal is generated for increasing the temperature of the portion 705 of the mouthpiece 33 in response to the control circuitry 18 determining that the temperature value is below a predetermined temperature. In this instance, the thermal element 710 may thus provide a heating action which may be implemented in any of the ways described above.

As discussed with respect to some embodiments, the temperature value may be indicative of an ambient temperature of the environment surrounding the aerosol provision system. Therefore, the thermal element 710 may be configured to heat the portion 705 of the mouthpiece 33 based on determining, with the temperature sensor 92, that an ambient temperature has fallen below a predetermined threshold. This may be particularly advantageous when operating the aerosol provision system in cold weather as the mouthpiece 33 may otherwise become uncomfortably cold for the user. Thus, by heating the mouthpiece 33 during cold ambient temperatures, the disclosed mouthpiece with its thermal element may improve the usability of the aerosol provision system.

According to some embodiments, the output signal may be generated for decreasing the temperature of the portion 705 of the mouthpiece 33 in response to the control circuitry 18 determining that the temperature is above a predetermined threshold. In this instance, the thermal element 710 provides a cooling action which may be implemented in any of the ways described above.

As discussed, the temperature value may be indicative of an ambient temperature of the environment surrounding the aerosol provision system. Therefore, the thermal element 710 may be configured to cool the portion 705 of the mouthpiece 33 based on determining, with the temperature sensor 92, that an ambient temperature has risen above a predetermined threshold. This may be particularly advantageous when operating the aerosol provision system in warm weather as the mouthpiece 33 may otherwise become uncomfortably hot for the user. Thus, by cooling the mouthpiece 33 during warm ambient temperatures, the disclosed mouthpiece with its thermal element may improve the usability of the aerosol provision system.

As noted above, it is envisaged that any thermal element integrated into the mouthpiece 33 may, in accordance with some embodiments, perform both heating and cooling of the mouthpiece 33. In accordance with some particular embodiments therefore, the thermal element 710 may be configured to both heat the portion 705 of the mouthpiece 33 based on the temperature value falling below a predetermined threshold, and cool the portion 705 of the mouthpiece 33 based on the temperature rising above a predetermined threshold. This arrangement is particularly advantageous in that the same component is used for both heating and cooling rather than using separate components. Thus, space within the mouthpiece may be used more economically and power consumed from the power source 16 from the aerosol provision system may be reduced. The control circuitry 18 is also simplified by using the same component for both functions.

Appreciably, the predetermined temperature thresholds described above may, in accordance with some embodiments, be the same temperature value. Alternatively, the predetermined thresholds in accordance with other embodiments may comprise different values such that there exists a temperature range in which the thermal element is not activated.

According to some embodiments, the aerosol provision system may appreciably comprise a reservoir 31 for storing aerosol-generating material. In this instance, the sensor data may be indicative of an amount of aerosol-generating material remaining in the reservoir 31, and the control circuitry 18 may be configured to determine, using the sensor data, whether the amount of aerosol-generating material remaining in the reservoir 31 is below a predetermined amount; and generate the output signal in response to determining that the amount of aerosol-generating material remaining in the reservoir 31 is below the predetermined amount. Therefore, the disclosed aerosol provision system may be able to provide feedback as to the level of remaining material in the reservoir 31 without the need for manual inspection or requiring separate visual cues such as LEDs. This also provides a more operationally efficient means of communicating the reservoir level to the user because the user may identify the reservoir level simply by contacting the mouthpiece 33 with their lips (as they would during normal use of the aerosol provision system) as opposed to removing any employed cartridge 2 for visual inspection or inspecting an LED or display from the aerosol provision system.

According to some embodiments, any provided control circuitry 18 may be further configured to: determine a composition of aerosol-generating material in the reservoir 31; generate a first output signal, in response to determining the composition matches a first composition, for operating the at least one thermal element 710; generate a second output signal, which is different from the first output signal, in response to determining the composition matches a second composition which is different from the first composition. In this way, the aerosol provision system may be configured to provide different thermal output depending on a detected composition of the aerosol-generating material in the reservoir 31. For example, the thermal output of the thermal element 710 may be configured to "match" the flavor of the detected composition. Thus, warmer flavors such as those comprising coffee or vanilla may correspond to the thermal element 710 providing heat to the mouthpiece 33. Similarly, cooler flavors such as those comprising mint or menthol may correspond to the thermal element 710 providing a cooling action on the mouthpiece 33. Therefore, the aerosol provision system may provide enhanced flavor perception to the user and simultaneously provide feedback relating to the user as to the flavor of the aerosol-generating material in the reservoir 31. Appreciably, it is noted that the aerosol provision system, in accordance with such embodiments, need not necessarily be required to generate vapor for the thermal feedback to be provided in this way such that the type of flavor can be identified by the user without inhaling the generated vapor.

According to some embodiments, the control circuitry 18 may be further configured to generate the output signal in response to determining that the aerosol provision system has been operated for a period of time which exceeds a predetermined threshold amount. In this way, the user may receive thermal feedback on how long the system has been operated for. The period of time may be measured in a number of different ways. For example, the period may be measured from when the system was last powered on, from the beginning of the day, from the time that the cartridge was inserted for the first time, etc.

When the predetermined threshold amount of time has passed, the thermal element 710 may be configured to provide heat to the mouthpiece 33 to inform the user that the threshold has been reached. Alternatively, a cooling action may be provided to transfer the same information.

Once the predetermined threshold amount of time has passed, the amount of heating/cooling may be increased to indicate that more time has passed since the threshold was passed.

As an alternative to waiting a predefined threshold amount of time before activating the thermal element 710, the amount of heating/cooling may be increased gradually from the start time, such that the amount of heating/cooling reaches a predefined level once the predefined threshold amount of time has passed. In this way, the user is provided with continuous feedback as to how long the device has been used for.

According to some embodiments, an aerosol provision device 4 may comprise the control circuitry 18 disclosed above.

According to some embodiments, there is provided a cartridge 2 for an aerosol provision system 300 comprising the cartridge 2 and an aerosol provision device 4, wherein the cartridge 2 comprises the mouthpiece 33 disclosed above.

Noting the above disclosures, it will be appreciated that these also provide for a corresponding method of controlling the temperature of a portion of a mouthpiece 33 from an aerosol provision system, the method comprising operating a thermal element from the mouthpiece 33 to control the temperature of the portion of the mouthpiece 33. In general terms, the method causes the thermal element 710 to provide feedback to the user on the operation of the aerosol provision system 300 via contact of the portion of the mouthpiece 33 with the user's lips/mouth. Furthermore, the use of the thermal element 710 allows for the improvement in user experience when using the aerosol provision system by changing the temperature of the portion of the mouthpiece according to a number of different factors. Thus, the use of a thermal element 710 on or in the mouthpiece 33 may help to increase the operability/usability of the aerosol provision system.

In some embodiments, the method may further comprise determining, using control circuitry 18 from the aerosol provision system 300, whether an output signal, for operating the at least one thermal element, should be generated; generating the output signal, using the control circuitry 18, in response to the control circuitry 18 determining that the output signal should be generated; and operating the thermal element 710 in response to the thermal element receiving the output signal.

In a similar fashion to the embodiments described with reference to the aerosol provision system 300 disclosed herein, the thermal element 710 may be able to be selectively activated in response to a control signal being generated by the control circuitry 18 in response to a number of different stimuli.

For instance, and in accordance with some embodiments, the method may further comprise receiving sensor data from a sensor 91 of the aerosol provision system 300; and processing the sensor data to determine whether the output signal should be generated. This provides a method for providing feedback to the user on the parameter being measured by the sensor 91 by way of activating the thermal element 710 based on a measurement of the parameter.

With reference to the foregoing disclosure, there has accordingly been described a number of different possible implementations for a thermal element for use in a mouthpiece 33 which is configured for use in any of the herein described aerosol provision devices or systems.

With respect to the above disclosure as well, and particularly where the at least one indicia is employed, it may be appreciated that the use of this at least one indicia may also consequentially provide for a mouthpiece which can appropriately indicate to the user an appropriate predetermined location for the user to place their lips when the user comes to place their lips around the mouthpiece. In this respect, the presence of this indication may facilitate the user locating their lips around the mouthpiece in an optimal position for receiving any aerosol from the aerosol outlet channel(s) 62 of the mouthpiece 33. In this respect as well, and in so far as the mouthpiece 33 may then further comprise the thermal element 710 for controlling the temperature of the portion 705 of the mouthpiece 33, any such at least one indicia may be particularly helpful for indicating to the user where their lips should be placed on the mouthpiece - i.e. at the location of the portion 705, such that the user's lips can then experience the temperature changes at the portion 705 during use of the mouthpiece 33.

This being the case, described herein may thus also be a mouthpiece 33 for an aerosol provision system 300, the mouthpiece 33 comprising: at least one aerosol outlet channel 62 for delivering an aerosol to a user of the mouthpiece 33; at least one external surface 33A;33B comprising a portion 705 which is configured for receiving a lip of a user of the mouthpiece 33 when aerosol is being delivered through the at least one aerosol outlet channel 62; and at least one indicia 706A;707 for identifying the location of the portion 705 of the mouthpiece 33.

Where any such mouthpiece 33 is employed in such instances, for use in indicating to the user an appropriate predetermined location for the user to place their lips when the user comes to place their lips around the mouthpiece, it will be appreciated that the mouthpiece in accordance with such embodiments need not necessarily comprise the herein described thermal element 710.

Appreciating the foregoing, there has accordingly been described a mouthpiece for an aerosol provision system, the mouthpiece comprising at least one aerosol outlet channel for delivering an aerosol to a user of the mouthpiece, wherein the mouthpiece comprises at least one thermal element for controlling the temperature of a portion of the mouthpiece.

There has also been described an aerosol provision system comprising the mouthpiece, wherein the aerosol outlet channel is configured to receive aerosol generated in the aerosol provision system.

There has also been described a cartridge for an aerosol provision system comprising the cartridge and an aerosol provision device, wherein the cartridge comprises the mouthpiece.

There has also been described a method of controlling the temperature of a portion of a mouthpiece from an aerosol provision system, wherein the method comprises: operating a thermal element from the mouthpiece to control the temperature of the portion of the mouthpiece.

There has also been described a mouthpiece for an aerosol provision system, the mouthpiece comprising: at least one aerosol outlet channel for delivering an aerosol to a user of the mouthpiece; at least one external surface comprising a portion which is configured for receiving a lip of a user of the mouthpiece when aerosol is being delivered through the at least one aerosol outlet channel; and at least one indicia for identifying the location of the portion of the mouthpiece.

There has also been described an aerosol provision system for generating an aerosol, comprising any of the mouthpieces as described above, wherein the aerosol outlet channel is configured to receive aerosol generated in the aerosol provision system.

There has also been described a cartridge for an aerosol provision system comprising the cartridge and an aerosol provision device configured to receive the cartridge, wherein the cartridge comprises any of the mouthpieces as described above.

There has also been described a mouthpiece 33 for an aerosol provision system 300. The mouthpiece 33 comprises at least one aerosol outlet channel 62 for delivering an aerosol to a user of the mouthpiece 33. The mouthpiece 33 comprises at least one thermal element 710 for controlling the temperature of a portion 705 of the mouthpiece 33. The portion 705 may comprise an exterior surface of the mouthpiece 33, and the thermal element 710 may be configured for heating and cooling the portion 705 of the mouthpiece 33. In this way, the thermal element 710 may allow for thermal feedback to be provide to the user, such as to their mouth, lips, or tongue whilst they are operating the aerosol provision system 300.

In order to address various issues and advance the art, this disclosure shows by way of illustration various embodiments in which the claimed invention(s) may be practiced. The advantages and features of the disclosure are of a representative sample of embodiments only, and are not exhaustive and/or exclusive. They are presented only to assist in understanding and to teach the claimed invention(s). It is to be understood that advantages, embodiments, examples, functions, features, structures, and/or other aspects of the disclosure are not to be considered limitations on the disclosure as defined by the claims or limitations on equivalents to the claims, and that other embodiments may be utilized and modifications may be made without departing from the scope of the claims. Various embodiments may suitably comprise, consist of, or consist essentially of, various combinations of the disclosed elements, components, features, parts, steps, means, etc. other than those specifically described herein, and it will thus be appreciated that features of the dependent claims may be combined with features of the independent claims in combinations other than those explicitly set out in the claims. The disclosure may include other inventions not presently claimed, but which may be claimed in future.

For instance, in terms of how any provided thermal element 710 may be powered (if it is employed at all), it will be appreciated that the thermal element 710 may be powered using either the power supply 16, or powered with its own power source (not shown in the Figures).

Equally, with regard to the positioning of any such thermal element 710 and/or sensor(s) 91 which may be employed herein, it will be appreciated that their locations may be provided anywhere in the aerosol provision system 300 as may be required to allow them to provide their required functionality.

Equally, where the aerosol provision system 300 comprises a cartridge 2 and an aerosol provision device 4, any provided sensor(s) 91 may be located in either the cartridge 2 or the aerosol provision device 4, as needed to allow the required functionality of the sensor.

For the sake of completeness as well, in respect of any thermal element 710, sensor(s), or switch(es) 91;92 employed herein in the aerosol provision device or system, it will be appreciated that any power or signals sent thereto may be provided using either a wired or wireless connection between the control circuitry 18 and the respective thermal element 710 / sensor or switch 91;92. In the particular embodiments shown in Figure 10, for instance, a wired connection is provided between the relevant thermal element 710 and the control circuitry 18, and which extends in the case of the component being located in the cartridge 2 across the interface end 54, via the contact electrodes 46 located on each of the aerosol provision device 4 and the cartridge 2 as shown in Figure 6B.

## Claims

1. A mouthpiece (33) for an aerosol provision system (1), the mouthpiece (33) comprising:
at least one aerosol outlet channel (62) for delivering an aerosol to a user of the mouthpiece (33); and
at least one thermal element (710) for controlling a temperature of a portion (705) of the mouthpiece, wherein the portion (705) comprises an exterior surface of the mouthpiece, wherein the thermal element (710) comprises at least one of a heater and/or cooler.

2. The mouthpiece (33) according to claim 1, wherein the thermal element (710) comprises an electrical resistor, located at the portion (705) of the mouthpiece (33), for heating the portion (705) of the mouthpiece (33); optionally wherein the electrical resistor comprises an electrical coil, located at the portion (705) of the mouthpiece (33), for heating the portion (705) of the mouthpiece (33).

3. The mouthpiece (33) according to claim 1, wherein the thermal element (710) comprises a Peltier module.

4. The mouthpiece (33) according to claim 1, wherein the portion (705) at least partially surrounds the aerosol outlet channel (62)

5. The mouthpiece (33) according to claim 1, wherein the portion (705) comprises at least one indicia for identifying a location of the portion optionally, wherein the at least one indicia comprises a protuberance or a recess; and/or
the at least one indicia comprises the portion (705) being colored differently from a color of a second portion (709) of the mouthpiece (33) which surrounds the portion (705) of the mouthpiece (33); and/or
the at least one indicia comprises the portion (705) being made of a different material from a material of a second portion (709) of the mouthpiece (33) which surrounds the portion (705) of the mouthpiece (33); optionally wherein the different material has a thermal conductivity which is greater than a thermal conductivity of the material of the second portion (709) of the mouthpiece (33).

6. The mouthpiece (33) according to claim 1, wherein the mouthpiece (33) comprises at least one external surface which is inclined towards an end of the mouthpiece (33);

7. The mouthpiece (33) according to claim 6, wherein at least one portion (705) of the thermal element (710) is located no more than 40mm from the end (52) of the mouthpiece comprising the aerosol outlet (60).

8. The mouthpiece according to claim 6, wherein the at least one external surface comprises:
a first external surface (33A) on a first side of the mouthpiece (33); and
a second external surface (33B) on a second side of the mouthpiece (33), wherein the second side is opposite the first side, optionally wherein at least one of the external surfaces comprises the portion (705) of the mouthpiece (33);
optionally wherein both the first external surface (33A) and the second external surface (33B) comprise the portion (705) of the mouthpiece (33).

9. The mouthpiece (33) according to claim 1, wherein the at least one aerosol outlet channel (62) is configured to be thermally insulated from the thermal element (710) and the portion (705) of the mouthpiece (33).

10. The mouthpiece (33) according to claim 1, further comprising a sensor (91) which is configured to generate sensor data for use in controlling an operation of the at least one thermal element (710); optionally wherein the sensor data comprises pressure data.

11. An aerosol provision system (1) for generating an aerosol, comprising the mouthpiece (33) according to claim 1, wherein the at least one aerosol outlet channel (62) is configured to receive aerosol generated in the aerosol provision system (1).

12. The aerosol provision system (1) according to claim 11, wherein the aerosol provision system (1) further comprises a cartridge (2) and an aerosol provision device (4) configured to receive the cartridge (2), wherein the cartridge (2) comprises the mouthpiece (33), or
wherein the mouthpiece (33) forms a part of the aerosol provision device (4).

13. The aerosol provision system (1) according to claim 11, further comprising control circuitry (18) which is configured to:
determine whether an output signal, for operating the at least one thermal element (710), should be generated; and
generate the output signal in response to determining that the output signal should be generated;
optionally further comprising a sensor for generating sensor data, wherein the control circuitry (18) is further configured to:
receive the sensor data from the sensor (91); and
process the sensor data to determine whether the output signal should be generated; optionally wherein the sensor (91) comprises a temperature sensor (91), and wherein the sensor data comprises a temperature value indicative of ambient temperature.

14. A cartridge (2), for an aerosol provision system (1) comprising an aerosol provision device (4) for receiving the cartridge (2), wherein the cartridge (2) comprises the mouthpiece (33) according to claim 1.

15. A method of controlling a temperature of a portion (705) of a mouthpiece (33) from an aerosol provision system (1), wherein the method comprises:
operating at least one thermal element (710) from the mouthpiece (33) to control the temperature of the portion (705) of the mouthpiece (33); wherein the portion (705) comprises an exterior surface of the mouthpiece (33), wherein the thermal element (710) comprises at least one of a heater and/or cooler.

## Patentansprüche

1. Mundstück (33) für ein Aerosolbereitstellungssystem (1), das Mundstück (33) umfassend:
mindestens einen Aerosolauslasskanal (62) zum Verabreichen eines Aerosols an einen Benutzer des Mundstücks (33); und
mindestens ein thermisches Element (710) zum Steuern einer Temperatur eines Abschnitts (705) des Mundstücks, wobei der Abschnitt (705) eine Außenfläche des Mundstücks umfasst, wobei das thermische Element (710) mindestens eines von einem Heizer und/oder einem Kühler umfasst.

2. Mundstück (33) nach Anspruch 1, wobei das thermische Element (710) einen elektrischen Widerstand umfasst, der sich an dem Abschnitt (705) des Mundstücks (33) befindet, um den Abschnitt (705) des Mundstücks (33) zu beheizen; wobei der elektrische Widerstand optional eine elektrische Spule, die sich an dem Abschnitt (705) des Mundstücks (33) befindet, zum Beheizen des Abschnitts (705) des Mundstücks (33) umfasst.

3. Mundstück (33) nach Anspruch 1, wobei das thermische Element (710) ein Peltier-Modul umfasst.

4. Mundstück (33) nach Anspruch 1, wobei der Abschnitt (705) den Aerosolauslasskanal (62) mindestens teilweise umgibt

5. Mundstück (33) nach Anspruch 1, wobei der Abschnitt (705) mindestens ein Erkennungsmerkmal zum Identifizieren einer Position des Abschnitts umfasst, wobei das mindestens eine Erkennungsmerkmal optional eine Erhebung oder eine Vertiefung umfasst; und/oder
das mindestens eine Erkennungsmerkmal umfasst, dass der Abschnitt (705) anders gefärbt ist als eine Farbe eines zweiten Abschnitts (709) des Mundstücks (33), der den Abschnitt (705) des Mundstücks (33) umgibt; und/oder
das mindestens eine Erkennungsmerkmal umfasst, dass der Abschnitt (705) aus einem anderen Material als das Material eines zweiten Abschnitts (709) des Mundstücks (33) gefertigt ist, der den Abschnitt (705) des Mundstücks (33) umgibt; wobei das unterschiedliche Material optional eine thermische Leitfähigkeit aufweist, die größer als die thermische Leitfähigkeit des Materials des zweiten Abschnitts (709) des Mundstücks (33) ist.

6. Mundstück (33) nach Anspruch 1, wobei das Mundstück (33) mindestens eine Außenfläche umfasst, die zu einem Ende des Mundstücks (33) hin geneigt ist;

7. Mundstück (33) nach Anspruch 6, wobei sich mindestens ein Abschnitt (705) des thermischen Elements (710) in einem Abstand von nicht mehr als 40 mm von dem Ende (52) des Mundstücks befindet, das den Aerosolauslass (60) umfasst.

8. Mundstück nach Anspruch 6, wobei die mindestens eine Außenfläche umfasst:
eine erste Außenfläche (33A) an einer ersten Seite des Mundstücks (33); und
eine zweite Außenfläche (33B) an einer zweiten Seite des Mundstücks (33), wobei die zweite Seite der ersten Seite entgegengesetzt ist, wobei optional mindestens eine der Außenflächen den Abschnitt (705) des Mundstücks (33) umfasst;
wobei optional sowohl die erste Außenfläche (33A) als auch die zweite Außenfläche (33B) den Abschnitt (705) des Mundstücks (33) umfassen.

9. Mundstück (33) nach Anspruch 1, wobei der mindestens eine Aerosolauslasskanal (62) dazu ausgebildet ist, von dem thermischen Element (710) und dem Abschnitt (705) des Mundstücks (33) thermisch isoliert zu sein.

10. Mundstück (33) nach Anspruch 1, weiter umfassend einen Sensor (91), der dazu ausgebildet ist, Sensordaten zur Verwendung beim Steuern eines Betriebs des mindestens einen thermischen Elements (710) zu erzeugen;
wobei die Sensordaten optional Druckdaten umfassen.

11. Aerosolbereitstellungssystem (1) zum Erzeugen eines Aerosols, umfassend das Mundstück (33) nach Anspruch 1, wobei der mindestens eine Aerosolauslasskanal (62) dazu ausgebildet ist, in dem Aerosolbereitstellungssystem (1) erzeugtes Aerosol aufzunehmen.

12. Aerosolbereitstellungssystem (1) nach Anspruch 11, wobei das Aerosolbereitstellungssystem (1) weiter eine Kartusche (2) und eine Aerosolbereitstellungsvorrichtung (4) umfasst, die dazu ausgebildet ist, die Kartusche (2) aufzunehmen, wobei die Kartusche (2) das Mundstück (33) umfasst, oder
wobei das Mundstück (33) einen Teil der Aerosolbereitstellungsvorrichtung (4) bildet.

13. Aerosolbereitstellungssystem (1) nach Anspruch 11, weiter umfassend eine Steuerschaltung (18), die zu Folgendem ausgebildet ist:
Bestimmen, ob ein Ausgabesignal zum Betreiben des mindestens einen thermischen Elements (710) erzeugt werden soll; und
Erzeugen des Ausgabesignals als Reaktion auf das Bestimmen, dass das Ausgabesignal erzeugt werden soll;
optional weiter umfassend einen Sensor zum Erzeugen von Sensordaten, wobei die Steuerschaltung (18) weiter ausgebildet zu Folgendem ist:
Empfangen der Sensordaten von dem Sensor (91); und
Verarbeiten der Sensordaten, um zu bestimmen, ob das Ausgabesignal erzeugt werden soll; wobei der Sensor (91) optional einen Temperatursensor (91) umfasst und wobei die Sensordaten einen Temperaturwert umfassen, der eine Umgebungstemperatur angibt.

14. Kartusche (2) für ein Aerosolbereitstellungssystem (1), das eine Aerosolbereitstellungsvorrichtung (4) zum Aufnehmen der Kartusche (2) umfasst, wobei die Kartusche (2) das Mundstück (33) nach Anspruch 1 umfasst.

15. Verfahren zum Steuern einer Temperatur eines Abschnitts (705) eines Mundstücks (33) von einem Aerosolbereitstellungssystem (1), das Verfahren umfassend:
Betreiben mindestens eines thermischen Elements (710) von dem Mundstück (33), um die Temperatur des Abschnitts (705) des Mundstücks (33) zu steuern; wobei der Abschnitt (705) eine Außenfläche des Mundstücks (33) umfasst, wobei das thermische Element (710) mindestens eines von einem Heizer und/oder einem Kühler umfasst.

## Revendications

1. Embout buccal (33) pour un système de fourniture d'aérosol (1), l'embout buccal (33) comprenant :
au moins un canal de sortie d'aérosol (62) permettant de délivrer un aérosol à un utilisateur de l'embout buccal (33) ; et au moins un élément thermique (710) permettant de commander une température d'une partie (705) de l'embout buccal, dans lequel la partie (705) comprend une surface extérieure de l'embout buccal, dans lequel l'élément thermique (710) comprend au moins l'un parmi un élément chauffant et/ou un refroidisseur.

2. Embout buccal (33) selon la revendication 1, dans lequel l'élément thermique (710) comprend une résistance électrique, située au niveau de la partie (705) de l'embout buccal (33), permettant de chauffer la partie (705) de l'embout buccal (33) ; facultativement dans lequel la résistance électrique comprend une bobine électrique, située au niveau de la partie (705) de l'embout buccal (33), permettant de chauffer la partie (705) de l'embout buccal (33).

3. Embout buccal (33) selon la revendication 1, dans lequel l'élément thermique (710) comprend un module Peltier.

4. Embout buccal (33) selon la revendication 1, dans lequel la partie (705) entoure au moins partiellement le canal de sortie d'aérosol (62)

5. Embout buccal (33) selon la revendication 1, dans lequel la partie (705) comprend au moins un repère permettant d'identifier un emplacement de la partie facultativement, dans lequel l'au moins un repère comprend une protubérance ou un évidement ; et/ou
l'au moins un repère comprend la partie (705) qui est colorée différemment d'une couleur d'une deuxième partie (709) de l'embout buccal (33) qui entoure la partie (705) de l'embout buccal (33) ; et/ou
l'au moins un repère comprend la partie (705) qui est composée d'un matériau différent d'un matériau d'une deuxième partie (709) de l'embout buccal (33) qui entoure la partie (705) de l'embout buccal (33) ; facultativement dans lequel le matériau différent présente une conductivité thermique qui est supérieure à une conductivité thermique du matériau de la deuxième partie (709) de l'embout buccal (33).

6. Embout buccal (33) selon la revendication 1, dans lequel l'embout buccal (33) comprend au moins une surface externe qui est inclinée en direction d'une extrémité de l'embout buccal (33);

7. Embout buccal (33) selon la revendication 6, dans lequel au moins une partie (705) de l'élément thermique (710) est située à une distance non supérieure à 40 mm de l'extrémité (52) de l'embout buccal comprenant la sortie d'aérosol (60).

8. Embout buccal selon la revendication 6, dans lequel l'au moins une surface externe comprend :
une première surface externe (33A) sur un premier côté de l'embout buccal (33) ; et
une deuxième surface externe (33B) sur un deuxième côté de l'embout buccal (33), dans lequel le deuxième côté est opposé au premier côté, facultativement dans lequel au moins l'une des surfaces externes comprend la partie (705) de l'embout buccal (33) ;
facultativement dans lequel à la fois la première surface externe (33A) et la deuxième surface externe (33B) comprennent la partie (705) de l'embout buccal (33).

9. Embout buccal (33) selon la revendication 1, dans lequel l'au moins un canal de sortie d'aérosol (62) est conçu pour être isolé thermiquement de l'élément thermique (710) et de la partie (705) de l'embout buccal (33).

10. Embout buccal (33) selon la revendication 1, comprenant en outre un capteur (91) qui est conçu pour générer des données de capteur destinées à être utilisées dans la commande d'un fonctionnement de l'au moins un élément thermique (710) ; facultativement dans lequel les données de capteur comprennent des données de pression.

11. Système de fourniture d'aérosol (1) permettant de générer un aérosol, comprenant l'embout buccal (33) selon la revendication 1, dans lequel l'au moins un canal de sortie d'aérosol (62) est conçu pour recevoir un aérosol généré dans le système de fourniture d'aérosol (1).

12. Système de fourniture d'aérosol (1) selon la revendication 11, dans lequel le système de fourniture d'aérosol (1) comprend en outre une cartouche (2) et un dispositif de fourniture d'aérosol (4) conçu pour recevoir la cartouche (2), dans lequel la cartouche (2) comprend l'embout buccal (33), ou
dans lequel l'embout buccal (33) forme une partie du dispositif de fourniture d'aérosol (4).

13. Système de fourniture d'aérosol (1) selon la revendication 11, comprenant en outre une circuiterie de commande (18) qui est conçue pour :
déterminer si un signal de sortie, permettant de faire fonctionner l'au moins un élément thermique (710), doit être généré ; et
générer le signal de sortie en réponse à la détermination que le signal de sortie doit être généré ;
comprenant en outre facultativement un capteur permettant de générer des données de capteur, dans lequel la circuiterie de commande (18) est en outre configurée pour :
recevoir les données de capteur du capteur (91) ; et
traiter les données de capteur pour déterminer si le signal de sortie doit être généré ou non ; facultativement dans lequel le capteur (91) comprend un capteur de température (91), et dans lequel les données de capteur comprennent une valeur de température indiquant une température ambiante.

14. Cartouche (2), pour un système de fourniture d'aérosol (1) comprenant un dispositif de fourniture d'aérosol (4) permettant de recevoir la cartouche (2), dans laquelle la cartouche (2) comprend l'embout buccal (33) selon la revendication 1.

15. Procédé de commande d'une température d'une partie (705) d'un embout buccal (33) à partir d'un système de fourniture d'aérosol (1), dans lequel le procédé comprend :
faire fonctionner au moins un élément thermique (710) depuis l'embout buccal (33) pour commander la température de la partie (705) de l'embout buccal (33) ; dans lequel la partie (705) comprend une surface extérieure de l'embout buccal (33), dans lequel l'élément thermique (710) comprend au moins l'un d'un élément chauffant et/ou d'un refroidisseur.
